# EUROPEAN PATENT SPECIFICATION

(11) **EP 1 889 609 B1**
(45) Date of publication and mention of the grant of the patent: **22.05.2019**
(21) Application number: 07013703.9
(22) Date of filing: 12.07.2007
(51) Int. Cl.: A61K 9/12, A61K 31/437, A61K 31/4745, A61P 15/00, A61P 15/02, A61P 17/00, A61P 17/08, A61P 17/12, A61P 17/16, A61P 17/18, A61P 31/12, A61P 35/00, A61P 43/00

(54) **Immune response modifier foam formulations**
Schaumformulierungen zur Modifikation der Immunantwort
Formulations de mousse modifiant la réponse immunitaire

(30) Priority: 18.07.2006 US 807669 P
(43) Date of publication of application: 20.02.2008
(73) Proprietor: Meda AB, 170 09 Solna (SE)
(72) Inventor: Vallejo, Janet A., Eagan, MN 55123 (US)
(74) Representative: FRKelly

(56) References cited:
- EP-A- 1 512 685
- WO-A-2005/020995
- WO-A2-03/080114
- US-A1- 2003 026 794
- US-A1- 2005 175 630
- US-A1- 2005 201 959
- HENGGE U ET AL: "Topical immunomodulators-progress towards treating inflammation, infection, and cancer" LANCET INFECTIOUS DISEASES, XX, US, vol. 1, no. 3, 1 October 2001 (2001-10-01), pages 189-198, XP004812198 ISSN: 1473-3099

## Description

### Background

There has been a major effort in recent years to discover new drug compounds that act by stimulating certain key aspects of the immune system, as well as by suppressing certain other aspects (see, e.g., U.S. Patent Nos. 6,039,969 and 6,200,592). These compounds, sometimes referred to as immune response modifiers (IRMs), appear to act through basic immune system mechanisms known as toll-like receptors to induce selected cytokine biosynthesis and may be used to treat a wide variety of diseases and conditions. For example, certain IRMs may be useful for treating viral diseases (e.g., human papilloma virus, hepatitis, herpes), neoplasias (e.g., basal cell carcinoma, squamous cell carcinoma, actinic keratosis), and TH₂-mediated diseases (e.g., asthma, allergic rhinitis, atopic dermatitis), and are also useful as vaccine adjuvants. Unlike many conventional anti-viral or anti-tumor compounds, the primary mechanism of action for IRMs is indirect, by stimulating the immune system to recognize and take appropriate action against a pathogen.

Many of the IRM compounds are imidazoquinoline amine derivatives (see, e.g., U.S. Pat. No. 4,689,338), but a number of other compound classes are now known as well (see, e.g., U.S. Pat. Nos. 5,446,153; 6,194,425; and 6,110,929).

Pharmaceutical compositions containing IRM compounds are disclosed in U.S. Patent Nos. 5, 238,944; 5,939,090; and 6,425,776; European Patent 0 394 026; and U.S. Patent Publication 2003/0199538. The IRM compound, 1-(2-methylpropyl)-1*H-*imidazo[4,5-c]quinolin-4-amine, also known as imiquimod, has been commercialized in a topical formulation, ALDARA, for the treatment of actinic keratosis, basal cell carcinoma, or anogenital warts associated with human papillomavirus.

The IRM compound, 2-methyl-1-(2-methylpropyl)-1H-imidazo[4,5-c][1,5]naphthyridin-4-amine, also known as sotirimod, has been described in EP 1 512 685, for the treatment of viral infections and the induction of cytokine biosynthesis.

Resiquimod (4-amino-α,α-dimethyl-2-ethoxymethyl-1H-imidazo[4,5-c]quinoline-1-ethanol), as described in EP 582 581 is a another member of the imidazoquinoline family of immune response modifiers.

Different topical formulations comprising IRM compounds are known.

WO 2005/020995 describes topical foam compositions comprising imiquimod for application to mucosal surfaces.

WO2003/080114 discloses the use of imiquimod as adjuvants for vaccines.

US 2005/175630 discloses immunogenic compositions for stimulating an immune response, comprising irradiated bacteria and e.g. imiquimod, formulated for mucosal delivery.

Pharmaceutical compositions for altering skin coloration comprising cytokines or imiquimod are known from US 2005/201959.

US 2003/026794 discloses a method of treating skin conditions by providing compositions containing enzymes to selectively remove specific layers of skin, optionally combined with additional active ingredients, e.g. imiquimod,

However, providing therapeutic benefit by topical application of an IRM compound for treatment of a particular condition at a particular location or of a particular tissue can be hindered by a variety of factors, such as, for example, chemical degradation of the IRM compound and/or other ingredients, and physical instability of the composition (e.g., separation of components, thickening, precipitation or agglomerization of active ingredient, and the like).

Therefore, there is a continuing need for new and/or improved imiquimod formulations.

### Summary

It has been found that imiquimod can be formulated as a pharmaceutical foam formulation.

Accordingly, the present invention provides pharmaceutical foam formulation. Generally, the formulation includes a therapeutically effective amount of imiquimod and isostearic acid.

In another aspect, the present invention also provides use of the formulation in a method of treating actinic keratosis. Generally, the use includes applying the formulation that includes a therapeutically effective amount of imiquimod and isostearic acid to the skin of a subject in need of such treatment.

In another aspect, the present invention also provides the use of the formulation in a method of treating basal cell carcinoma. Generally, the use includes applying the formulation that includes a therapeutically effective amount of imiquimod and a isostearic acid to the skin of a subject in need of such treatment.

In another aspect, the present invention also provides the use of the formulation in a method of treating anogential warts. Generally, the use includes applying the formulation that includes a therapeutically effective amount of imiquimod and isostearic acid to the skin of a subject in need of such treatment.

In another aspect, the present invention also provides the use of the formulation in a method of treating photodamaged skin. Generally, the use includes applying the formulation that includes a therapeutically effective amount of imiquimod and isostearicacid to the skin of a subject in need of such treatment.

In another aspect, the present invention also provides the use of the formulation in amethod of treating cervical dysplasia. Generally, the use includes applying the formulation that includes a therapeutically effective amount of imiquimod and isostearic acid to the skin of a subject in need of such treatment.

In another aspect, the present invention also provides the use of the formulation in a method of treating viral diseases, especially herpes. Generally, the use includes applying the formulation that includes a therapeutically effective amount of imiquimod and isostearic acid to the skin of a subject in need of such treatment.

In another aspect, the present invention also provides the use of the formulation in a method of treating cutaneous metastases. Generally, the use includes applying the formulation that includes a therapeutically effective amount of imiquimod and isostearic acid to the skin of a subject in need of such treatment.

In another aspect, the present invention also provides the use of the formulation in a method of treating dermal lesions caused by envenomation. Generally, the useincludes applying the formulation that includes a therapeutically effective amount of imiquimod and isostearic acid to the skin of a subject in need of such treatment.

In another aspect, the present invention also provides the use of the formulation in a method of treating keratoacanthoma. Generally, the use includes applying the formulation that includes a therapeutically effective amount of imiquimod and isostearic acid to the skin of a subject in need of such treatment.

Various other features and advantages of the present invention should become readily apparent with reference to the following detailed description, examples, claims and appended drawings. In several places throughout the specification, guidance is provided through lists of examples. In each instance, the recited list serves only as a representative group and should not be interpreted as an exclusive list.

### Detailed Description of Illustrative Embodiments of the Invention

It has been found that imiquimod may be formulated in an emulsion-based foam. Generally, the foam formulation includes a therapeutically effective amount of imiquimod and isostearic acid.

As used herein, "a," "an," "the," "at least one," and "one or more" are used interchangeably. Thus, for example, a formulation comprising "a" fatty acid can be interpreted to mean that the formulation includes at least one fatty acid.

Also herein, the recitations of numerical ranges by endpoints include all numbers subsumed within that range (e.g., 1 to 5 includes 1, 1.5, 2, 2.75, 3, 3.80, 4, 5, etc.).

Unless otherwise indicated, reference to a compound can include the compound in any pharmaceutically acceptable form, including any isomer (e.g., diastereomer or enantiomer), salt, solvate, polymorph, and the like. In particular, if a compound is optically active, reference to the compound can include each of the compound's enantiomers as well as racemic and scalemic mixtures of the enantiomers.

The formulation includes the imidazoquinoline amine 1-(2-methylpropyl)-1*H-*imidazo[4,5-*c*]quinolin-4-amine, also referred to as imiquimod, the synthesis of which is described at, for example, U.S. Patent No. 4,689,338, Example 99.

The amount of imiquimod present in a composition of the invention will be an amount effective to treat, prevent the recurrence of, or promote immunity to the targeted disease state, or to improve skin quality. The total amount of imiquimod can be at least 0.1 percent by weight, and no more than 9 percent by weight. In certain embodiments, the total amount of imiquimod can be at least 0.5 percent by weight, and no more than 9 percent by weight, based on the total weight of the composition (unless otherwise indicated, all percentages provided herein are weight/weight with respect to the total weight of the composition), although in some embodiments the composition may contain an amount of imiquimod outside of this range.. For example, the composition may include imiquimod at a concentration of 0,1%, 0,5% 1%, 2%, 3%, 4%, 5%, 6%, 7%, 8%or 9%.

The foam compositions of the invention may include one or more additional excipients such as, for example, a fatty acid, a preservative, a thickener, an emulsifier, a solubilizing agent, an emollient, or a humectant.

The formulations of the invention include one or more fatty acids. As used herein, the term "fatty acid" means a carboxylic acid, either saturated or unsaturated having 6 to 28 carbon atoms, such as, for example, from 10 to 22 carbon atoms. Fatty acids suitable for use in the formulations described herein include those that can aid in solubilizing imiquimod. Suitable fatty acids include, for example, isostearic acid, oleic acid, myristic acid, palmitic acid, palmitoleic acid, margaric acid, stearic acid, linoleic acid, linolenic acid, or mixtures thereof. In some embodiments, the formulation includes, for example, isostearic acid, oleic acid, or a mixture thereof. In one particular embodiment, the formulation includes isostearic acid.

The fatty acid is present in the formulation in an amount sufficient to solubilize imiquimod. In some embodiments, the total amount of fatty acid, is at least 0.05% by weight, at least 1.0% by weight, at least 3.0% by weight, at least 5.0%, at least 10%, at least 15%, or at least 25%, based on the total weight of the formulation. In certain embodiments, the total amount of fatty acid is at most 40% by weight, at most 30% by weight, at most 15% by weight, or at most 10% by weight, based on the total weight of the formulation.

For certain embodiments of the invention, the formulation further includes a propellant. In certain embodiments, the propellant is a hydrocarbon propellant. Suitable hydrocarbon propellants include, for example, lower alkanes, such as for example, propane, isobutane and butane. Any combination of suitable propellants can be included in the formulation. For example, the formulation can include a combination of propane and butane. One embodiment includes a 50:50 combination of propane and butane. Another embodiment includes a 10:90 combination of propane and butane. Another embodiment includes a 15:85 combination of propane and butane.

The total amount of propellant can be from about 2% to about 25%, although in some embodiments, the formulation can include a total amount of propellant outside of this range. In one embodiment, the total amount of propellant is about 5%. In another embodiment, the total amount of propellant is about 10%. In still another embodiment, the total amount of propellant is about 15%.

For certain embodiments of the invention, the formulation further includes a preservative system. The preservative system includes one or more compounds that inhibit microbial growth (e.g., fungal and bacterial growth) within the formulation (for example, during manufacturing and use). The preservative system will generally include at least one preservative compound, such as, for example, methylparaben, ethylparaben, propylparaben, butylparaben, benzyl alcohol, phenoxyethanol, and sorbic acid or derivatives of sorbic acid such as esters and salts. Various combinations of these compounds can be included in the preservative system. In some embodiments of the invention, the preservative system includes methylparaben, propylparaben and benzyl alcohol. In other embodiments of the invention, the preservative system includes methylparaben and benzyl alcohol.

In some embodiments of the invention, the preservative system is present in an amount of at least 0.01% by weight, such as for example, at least 0.02%, at least 0.03%, at least 0.04%, and at least 0.05%, by weight based on the total weight of the formulation. In other embodiments of the invention the preservative system is present in an amount of at most 10%, preferably at most 3%, such as for example, at most 7%, at most 5%, at most 2.5%, at most 2.0%, at most 1.0%, at most 0.5%, at most 0.4%, at most 0.3%, and at most 0.2%, by weight based on the total weight of the formulation.

For certain embodiments of the invention, the formulation further includes an emulsifier. Suitable emulsifiers include non-ionic surfactants such as, for example, polysorbate 60, sorbitan monostearate, polyglyceryl-4 oleate, polyoxyethylene(4) lauryl ether, poloxamers, and sorbitan trioleate. In certain embodiments, the emulsifier is chosen from polysorbate 60, sorbitan monostearate, and mixtures thereof.

If included, the emulsifier is generally present in an amount of 0.1% to 10% by weight of total formulation weight, for example, from 0.5% to 5.0% by weight, and from 0.75% to 4.0% by weight. In certain embodiments, the amount of the emulsifier, if used, is present in an amount of at least 0.1% by weight, at least 0.5% by weight, at least 0.75% by weight, at least 1.0% by weight, at least 2.5% by weight, at least 3.5% by weight, at least 4.0% by weight, or at least 5.0% by weight, based on the total weight of the formulation. In certain embodiments, the amount of the emulsifier, if used, is present in an amount of at most 10% by weight, at most 5.0% by weight, or at most 3.5% by weight, based on the total weight of the formulation.

For certain embodiments of the invention, the formulation further includes a viscosity-enhancing agent. Examples of suitable viscosity enhancing agents include long chain alcohols, for example, cetyl alcohol, stearyl alcohol, cetearyl alcohol; cellulose ethers such as hydroxypropylmethylcellulose, hydroxyethylcellulose, hydroxypropylcellulose, and carboxymethylcellulose; polysaccharide gums such as xanthan gum; and homopolymers and copolymers of acrylic acid crosslinked with allyl sucrose or allyl pentaerythriol such as those polymers designated as carbomers in the United States Pharmacopoeia. In certain embodiments, the viscosity enhancing agent is xanthan gum.

In certain embodiments, the amount of the viscosity enhancing agent, when used, is at least 0.1% by weight, at least 0.2% by weight, at least 0.5% by weight, at least 0.6% by weight, at least 0.7% by weight, at least 0.9% by weight, or at least 1.0% by weight, based on the total weight of the formulation. In certain embodiments, the amount of the viscosity-enhancing agent, when used, is at most 10% by weight, at most 5.0% by weight, at most 3.0% by weight, at most 2.0% by weight, or at most 1.5% by weight, based on the total weight of the formulation.

For certain embodiments of the invention, the formulation further includes at least one emollient. Examples of suitable emollients include, but are not limited to, long chain alcohols, for example, cetyl alcohol, stearyl alcohol, cetearyl alcohol; fatty acid esters, for example, isopropyl mysristate, isopropyl palmitate, diisopropyl dimer dilinoleate; medium-chain (e.g., 8 to 14 carbon atoms) triglycerides, for example, caprylic/capric triglyceride; cetyl esters; hydrocarbons of 8 or more carbon atoms, for example, light mineral oil, white petrolatum; and waxes, for example, beeswax. Various combinations of such emollients can be used if desired. In certain embodiments, the emollient is chosen from cetyl alcohol, stearyl alcohol, petolatum, and mixtures thereof.

In certain embodiments, the amount of the emollient is at least 1.0% by weight, at least 3.0% by weight, at least 5.0% by weight, or at least 10% by weight, based on the total weight of the formulation. In certain embodiments, the amount of emollient is at most 30% by weight, at most 15% by weight, or at most 10% by weight, based on the total weight of the formulation.

In some embodiments, formulations of the invention are oil-in-water emulsions. The water used in these formulations is typically purified water

Optionally, a formulation of the invention can include additional pharmaceutically acceptable excipients such as humectants, such as for example, glycerin; chelating agents, such as for example, ethylenediaminetetraacetic acid; and pH adjusting agents, such as for example, potassium hydroxide or sodium hydroxide. In certain embodiments, the formulation includes glycerin.

In some instances, a single ingredient can perform more than one function in a formulation. For example, cetyl alcohol can serve as both an emollient and a viscosity enhancer.

Conditions that may be treated by administering imiquimod in a foam composition of the invention include, but are not limited to:
(a) viral diseases such as, for example, diseases resulting from infection by an adenovirus, a herpesvirus (e.g., HSV-I, HSV-II, CMV, or VZV), a poxvirus (e.g., an orthopoxvirus such as variola or vaccinia, or molluscum contagiosum), a picornavirus (e.g., rhinovirus or enterovirus), an orthomyxovirus (e.g., influenzavirus), a paramyxovirus (e.g., parainfluenzavirus, mumps virus, measles virus, and respiratory syncytial virus (RSV)), a coronavirus (e.g., SARS), a papovavirus (e.g., papillomaviruses, such as those that cause genital warts, common warts, or plantar warts), a hepadnavirus (e.g., hepatitis B virus), a flavivirus (e.g., hepatitis C virus or Dengue virus), or a retrovirus (e.g., a lentivirus such as HIV);
(b) bacterial diseases such as, for example, diseases resulting from infection by bacteria of, for example, the genus Escherichia, Enterobacter, Salmonella, Staphylococcus, Shigella, Listeria, Aerobacter, Helicobacter, Klebsiella, Proteus, Pseudomonas, Streptococcus, Chlamydia, Mycoplasma, Pneumococcus, Neisseria, Clostridium, Bacillus, Corynebacterium, Mycobacterium, Campylobacter, Vibrio, Serratia, Providencia, Chromobacterium, Brucella, Yersinia, Haemophilus, or Bordetella;
(c) other infectious diseases, such chlamydia, fungal diseases including but not limited to candidiasis, aspergillosis, histoplasmosis, cryptococcal meningitis, or parasitic diseases including but not limited to malaria, pneumocystis carnii pneumonia, leishmaniasis, cryptosporidiosis, toxoplasmosis, and trypanosome infection;
(d) neoplastic diseases, such as intraepithelial neoplasias, cervical dysplasia, actinic keratosis, keratoacanthoma, basal cell carcinoma, squamous cell carcinoma, cutaneous metastases, renal cell carcinoma, Kaposi's sarcoma, melanoma, leukemias including but not limited to myelogeous leukemia, chronic lymphocytic leukemia, multiple myeloma, non-Hodgkin's lymphoma, cutaneous T-cell lymphoma, B-cell lymphoma, and hairy cell leukemia, and other cancers;
(e) T_{H}2-mediated, atopic diseases, such as atopic dermatitis or eczema, eosinophilia, asthma, allergy, allergic rhinitis, and Ommen's syndrome;
(f) certain autoimmune diseases such as systemic lupus erythematosus, essential thrombocythaemia, multiple sclerosis, discoid lupus, alopecia areata; and
(g) diseases associated with wound repair such as, for example, inhibition of keloid formation and other types of scarring (e.g., enhancing wound healing, including chronic wounds).
(h) dermal conditions such as, for example, photo damaged skin, improvement of skin quality, removing a tattoo, or dermal lesions caused by envenomation.

Skin quality is continuously affected by various factors including, for example, humidity, UV-light, cosmetics, aging, diseases, stress, cigarette smoking, and eating habits

Skin treated by practicing the invention can include facial skin, skin on the neck, hands, arms, legs, or torso, and skin of other body regions.

Improving skin quality includes reversing, slowing the progression of, or preventing skin changes associated with natural or innate aging. As used herein, "prevent" and variations thereof refer to any degree of delaying the onset of skin changes. For example, improving skin quality includes the reversal, slowing the progression of, or prevention of skin changes associated with sun damage or photoaging - i.e., skin changes associated with exposure to sunlight or other forms of actinic radiation (for example, such as UV radiation and tanning booths). As another example, improving skin quality also can include reversing, slowing the progression of, or preventing skin changes resulting from extrinsic factors, including, but not limited to, radiation, air pollution, wind, cold, dampness, heat, chemicals, smoke, cigarette smoking, and combinations thereof.

Improving skin quality also can include reversing, preventing or reducing scarring the can result, for example, from certain skin conditions (e. g. acne), infections (i. e. , leishmaniasis), or injury (e. g., abrasions, punctures, lacerations, or surgical wounds).

Skin changes treatable by practicing the invention include, for example, wrinkles (including, but not limited to, human facial wrinkles), deepening of skin lines, thinning of skin, reduced scarring, yellowing of the skin, mottling, hyperpigmentation, appearance of pigmented and/or non-pigmented age spots, leatheriness, loss of elasticity, loss of recoilability, loss of collagen fibers, abnormal changes in the elastic fibers, deterioration of small blood vessels of the dermis, formation of spider veins, and combinations thereof.

Additionally, the foam compositions may be useful for delivery of imiquimod as a topical vaccine adjuvant for use in conjunction with any material that raises either humoral and/or cell-mediated immune response, such as, for example, live viral, bacterial, or parasitic immunogens; inactivated viral, tumor-derived, protozoal, organism-derived, fungal, or bacterial immunogens, toxoids, toxins; self-antigens; polysaccharides; proteins; glycoproteins; peptides; cellular vaccines; DNA vaccines; autologous vaccines; recombinant proteins; glycoproteins; peptides; and the like, for use in connection with, for example, BCG, cholera, plague, typhoid, hepatitis A, hepatitis B, hepatitis C, influenza A, influenza B, parainfluenza, polio, rabies, measles, mumps, rubella, yellow fever, tetanus, diphtheria, hemophilus influenza b, tuberculosis, meningococcal and pneumococcal vaccines, adenovirus, HIV, chicken pox, cytomegalovirus, dengue, feline leukemia, fowl plague, HSV-1 and HSV-2, hog cholera, Japanese encephalitis, respiratory syncytial virus, rotavirus, papilloma virus, yellow fever, and Alzheimer's Disease.

The methods of the present invention may be performed on any suitable subject. Suitable subjects include but are not limited to animals such as, for example, humans, non-human primates, poultry, fowl, rodents, dogs, cats, horses, pigs, sheep, goats, or cows.

### Examples

The following examples have been selected merely to further illustrate features, advantages, and other details of the invention. It is to be expressly understood, however, that while the examples serve this purpose, the particular materials and amounts used as well as other conditions and details are not to be construed in a matter that would unduly limit the scope of this invention.

The formulations shown in Tables 1 to 4 below were prepared using the following general method.

Imiquimod/isostearic acid/benzyl alcohol premix preparation: Imiquimod was combined with isostearic acid and mixed with heating (about 55°C) until the bulk of the imiquimod was dissolved. Benzyl alcohol was added and mixing was continued until all of the imiquimod was in solution.

Oil phase preparation: Cetyl alcohol, stearyl alcohol, white petrolatum, polysorbate 60, and sorbitan monostearate were added to the imiquimod/isostearic acid/benzyl alcohol premix and mixed with heating (about 55°C) until all components were dissolved.

Aqueous phase preparation: Methylparaben and propylparaben were combined with water and a portion (about 50%) of the glycerin and mixed with heating (about 55°C) until the parabens were dissolved. Separately, a dispersion of xanthan gum was prepared by combining xanthan gum with the remaining portion of glycerin and mixing until the xanthan gum was dispersed. The xanthan gum dispersion was slowly added with mixing to the parabens solution while maintaining the temperature at about 55°C. Mixing was continued until the xanthan gum was fully dispersed.

Phase combination: The aqueous phase was added to the oil phase at about 55°C. The mixture was homogenized for a minimum of 15 minutes. The resulting emulsion was cooled to ambient temperature and then placed in a glass jar. Tables 1 and 2 below show the composition (percentage weight-by-weight basis) of the formulations prior to the addition of the propellant.

Addition of propellant: Emulsion was placed in a plastic-coated glass vial. An aerosol valve, either a continuous valve or a metered-dose valve, was crimped onto the vial. The vial was charged with propellant using a pressure burette with a nitrogen headspace. The vial was then agitated to disperse the emulsion in the propellant. Tables 3 and 4 below show the composition (percentage weight-by-weight basis) of the formulations after addition of propellant.

| **Table 1** | | | | | | |
|---|---|---|---|---|---|---|
| **Formulations Prior to the Addition of Propellant** | | | | | | |
| **Ingredient** | **No 1** | **No 2** | **No 3** | **No 4** | **No 5** | **No 6** |
| Imiquimod | 5.88 | 5.88 | 5.56 | 5.26 | 5 | 5 |
| Isostearic acid | 29.41 | 29.41 | 27.78 | 26.32 | 25 | 25 |
| Benzyl alcohol | 2.35 | 2.35 | 2.22 | 2.11 | 2 | 2 |
| Polysorbate 60 | 4.00 | 4.00 | 3.78 | 3.58 | 3.4 | 3.4 |
| Sorbitan monostearate | 0.71 | 0.71 | 0.67 | 0.63 | 0.6 | 0.6 |
| Xanthan gum | 0.59 | 0.59 | 0.56 | 0.53 | 0.5 | 0.5 |
| Methylparaben | 0.24 | 0.24 | 0.22 | 0.21 | 0.2 | 0.2 |
| Propylparaben | 0.02 | 0.02 | 0.02 | 0.02 | 0.02 | 0.02 |
| Cetyl alcohol | 2.59 | 2.59 | 2.44 | 2.32 | 2.2 | 2.2 |
| Stearyl alcohol | 3.65 | 3.65 | 3.44 | 3.26 | 3.1 | 3.1 |
| Petrolatum | 3.53 | 3.53 | 3.33 | 3.16 | 3 | 3 |
| Glycerin | 2.35 | 2.35 | 2.22 | 2.11 | 2 | 2 |
| Water | 44.68 | 44.68 | 47.76 | 50.51 | 52.98 | 52.98 |

| **Table 2** | | | | | | |
|---|---|---|---|---|---|---|
| **Formulations Prior to the Addition of Propellant** | | | | | | |
| **Ingredient** | **No 7** | **No 8** | **No 9** | **No 10** | **No 11** | **No 12** |
| Imiquimod | 5.56 | 5.56 | 5.56 | 5.56 | 5.56 | 5.56 |
| Isostearic acid | 13.95 | 13.95 | 13.95 | 13.95 | 13.95 | 13.95 |
| Benzyl alcohol | 2.22 | 2.22 | 2.22 | 2.22 | 2.22 | 2.22 |
| Polysorbate 60 | 1.90 | 1.90 | 1.90 | 1.90 | 1.90 | 1.90 |
| Sorbitan monostearate | 2.00 | 2.00 | 2.00 | 2.00 | 2.00 | 2.00 |
| Xanthan gum | 0.15 | 0.15 | 0.15 | 0.15 | 0.15 | 0.15 |
| Methylparaben | 0.33 | 0.33 | 0.33 | 0.22 | 0.22 | 0.22 |
| Propylparaben | 0.03 | 0.03 | 0.03 | 0.02 | 0.02 | 0.02 |
| Cetyl alcohol | 1.30 | 1.30 | 1.30 | 1.30 | 1.30 | 1.30 |
| Stearyl alcohol | 1.80 | 1.80 | 1.80 | 1.80 | 1.80 | 1.80 |
| Petrolatum | 1.75 | 1.75 | 1.75 | 1.75 | 1.75 | 1.75 |
| Glycerin | 3.10 | 3.10 | 3.10 | 3.10 | 3.10 | 3.10 |
| Water | 65.91 | 65.91 | 65.91 | 66.03 | 66.03 | 66.03 |

| **Table 3** | | | | | | |
|---|---|---|---|---|---|---|
| **Formulations After the Addition of Propellant** | | | | | | |
| **Ingredient** | **No 1** | **No 2** | **No 3** | **No 4** | **No 5** | **No 6** |
| Imiquimod | 5 | 5 | 5 | 5 | 4.25 | 4.75 |
| Isostearic acid | 25 | 25 | 25 | 25 | 21.25 | 23.75 |
| Benzyl alcohol | 2 | 2 | 2 | 2 | 1.7 | 1.9 |
| Polysorbate 60 | 3.4 | 3.4 | 3.4 | 3.4 | 2.89 | 3.23 |
| Sorbitan monostearate | 0.6 | 0.6 | 0.6 | 0.6 | 0.51 | 0.57 |
| Xanthan gum | 0.5 | 0.5 | 0.5 | 0.5 | 0.43 | 0.48 |
| Methylparaben | 0.2 | 0.2 | 0.2 | 0.2 | 0.17 | 0.19 |
| Propylparaben | 0.02 | 0.02 | 0.02 | 0.02 | 0.02 | 0.02 |
| Cetyl alcohol | 2.2 | 2.2 | 2.2 | 2.2 | 1.87 | 2.09 |
| Stearyl alcohol | 3.1 | 3.1 | 3.1 | 3.1 | 2.64 | 2.95 |
| Petrolatum | 3 | 3 | 3 | 3 | 2.55 | 2.85 |
| Glycerin | 2 | 2 | 2 | 2 | 1.7 | 1.9 |
| Water | 37.98 | 37.98 | 42.98 | 47.98 | 45.02 | 50.32 |
| 50:50 Propane:butane | 15 | 0 | 10 | 5 | 15 | 5 |
| 10:90 Propane:butane | 0 | 15 | 0 | 0 | 0 | 0 |

| **Table 4** | | | | | | |
|---|---|---|---|---|---|---|
| **Formulations After the Addition of Propellant** | | | | | | |
| **Ingredient** | **No 7** | **No 8** | **No 9** | **No 10** | **No 11** | **No 12** |
| Imiquimod | 5.00 | 5.00 | 5.00 | 5.00 | 5.00 | 5.00 |
| Isostearic acid | 12.56 | 12.56 | 12.56 | 12.56 | 12.56 | 12.56 |
| Benzyl alcohol | 2.00 | 2.00 | 2.00 | 2.00 | 2.00 | 2.00 |
| Polysorbate 60 | 1.71 | 1.71 | 1.71 | 1.71 | 1.71 | 1.71 |
| Sorbitan monostearate | 1.80 | 1.80 | 1.80 | 1.80 | 1.80 | 1.80 |
| Xanthan gum | 0.14 | 0.14 | 0.14 | 0.14 | 0.14 | 0.14 |
| Methylparaben | 0.30 | 0.30 | 0.30 | 0.20 | 0.20 | 0.20 |
| Propylparaben | 0.03 | 0.03 | 0.03 | 0.02 | 0.02 | 0.02 |
| Cetyl alcohol | 1.17 | 1.17 | 1.17 | 1.17 | 1.17 | 1.17 |
| Stearyl alcohol | 1.62 | 1.62 | 1.62 | 1.62 | 1.62 | 1.62 |
| Petrolatum | 1.58 | 1.58 | 1.58 | 1.58 | 1.58 | 1.58 |
| Glycerin | 2.79 | 2.79 | 2.79 | 2.79 | 2.79 | 2.79 |
| Water | 59.30 | 59.30 | 59.30 | 59.41 | 59.41 | 59.41 |
| 50:50 Propane:butane | 10 | 0 | 0 | 10 | 0 | 0 |
| 15:85 Propane:butane | 0 | 10 | 0 | 0 | 10 | 0 |
| Isobutane | 0 | 0 | 10 | 0 | 0 | 10 |

The formulations shown in Tables 5 and 6 below were prepared using the following general method.

Imiquimod/isostearic acid premix preparation: Imiquimod was combined with isostearic acid and mixed with heating (about 55°C) until the bulk of the imiquimod was dissolved until all of the imiquimod was in solution.

Oil phase preparation: Cetyl alcohol, stearyl alcohol, white petrolatum, polysorbate 60, and sorbitan monostearate were added to the imiquimod/isostearic acid premix and mixed with heating (about 55°C) until all components were dissolved.

Aqueous phase preparation: Methylparaben and propylparaben were combined with water and a portion (about 50%) of the glycerin and mixed with heating (about 55°C) until the parabens were dissolved. Separately, a dispersion of xanthan gum was prepared by combining xanthan gum with the remaining portion of glycerin and mixing until the xanthan gum was dispersed. The xanthan gum dispersion was slowly added with mixing to the parabens solution while maintaining the temperature at about 55°C. Mixing was continued until the xanthan gum was fully dispersed.

Phase combination: The aqueous phase was added to the oil phase at about 55°C. The mixture was homogenized for a minimum of 15 minutes. The resulting emulsion was cooled to ambient temperature and then placed in a glass jar. Table 5 below shows the composition (percentage weight-by-weight basis) of the formulations prior to the addition of the propellant.

Addition of propellant: Emulsion was placed in a plastic-coated glass vial. An aerosol valve, either a continuous valve or a metered-dose valve, was crimped onto the vial. The vial was charged with propellant using a pressure burette with a nitrogen headspace. The vial was then agitated to disperse the emulsion in the propellant. Table 6 below shows the composition (percentage weight-by-weight basis) of the formulations after addition of propellant.

| **Table 5** | | | |
|---|---|---|---|
| **Formulations Prior to the Addition of Propellant** | | | |
| **Ingredient** | **No 13** | **No 14** | **No 15** |
| Imiquimod | 5.56 | 5.56 | 5.56 |
| Isostearic acid | 13.95 | 13.95 | 13.95 |
| Polysorbate 60 | 1.90 | 1.90 | 1.90 |
| Sorbitan monostearate | 2.00 | 2.00 | 2.00 |
| Xanthan gum | 0.15 | 0.15 | 0.15 |
| Methylparaben | 0.33 | 0.33 | 0.33 |
| Propylparaben | 0.03 | 0.03 | 0.03 |
| Cetyl alcohol | 1.30 | 1.30 | 1.30 |
| Stearyl alcohol | 1.80 | 1.80 | 1.80 |
| Petrolatum | 1.75 | 1.75 | 1.75 |
| Glycerin | 3.10 | 3.10 | 3.10 |
| Water | 68.13 | 68.13 | 68.13 |

| **Table 6** | | | |
|---|---|---|---|
| **Formulations After the Addition of Propellant** | | | |
| **Ingredient** | **No 13** | **No 14** | **No 15** |
| Imiquimod | 5.00 | 5.00 | 5.00 |
| Isostearic acid | 12.56 | 25.00 | 25.00 |
| Polysorbate 60 | 1.71 | 3.40 | 3.40 |
| Sorbitan monostearate | 1.80 | 0.60 | 0.60 |
| Xanthan gum | 0.14 | 0.50 | 0.50 |
| Methylparaben | 0.30 | 0.20 | 0.20 |
| Propylparaben | 0.03 | 0.02 | 0.02 |
| Cetyl alcohol | 1.17 | 2.20 | 2.20 |
| Stearyl alcohol | 1.62 | 3.10 | 3.10 |
| Petrolatum | 1.58 | 3.00 | 3.00 |
| Glycerin | 2.79 | 2.00 | 2.00 |
| Water | 61.30 | 37.98 | 42.98 |
| 50:50 Propane:butane | 10 | 0 | 0 |
| 15:85 Propane:butane | 0 | 10 | 0 |
| Isobutane | 0 | 0 | 10 |

The formulations shown in Tables 7 and 8 below were prepared using the following general method.

Imiquimod/isostearic acid/benzyl alcohol premix preparation: Imiquimod was combined with isostearic acid and mixed with heating (about 55°C) until the bulk of the imiquimod was dissolved. Benzyl alcohol was added and mixing was continued until all of the imiquimod was in solution.

Oil phase preparation: Polysorbate 60 and sorbitan monostearate were added to the imiquimod/isostearic acid/benzyl alcohol premix and mixed with heating (about 55°C) until all components were dissolved.

Aqueous phase preparation: Methylparaben and propylparaben, if included, were combined with water and mixed with heating (about 55°C) until the parabens were dissolved. Xanthan gum was added and the aqueous phase was mixed at about 55°C until the xanthan gum was fully dispersed.

Phase combination: The aqueous phase was added to the oil phase at about 55°C. The mixture was homogenized for a minimum of 15 minutes. The resulting emulsion was cooled to ambient temperature and then placed in a glass jar. Table 7 below shows the composition (percentage weight-by-weight basis) of the formulations prior to the addition of the propellant.

Addition of propellant: Emulsion was placed in a plastic-coated glass vial. An aerosol valve, either a continuous valve or a metered-dose valve, was crimped onto the vial. The vial was charged with propellant using a pressure burette with a nitrogen headspace. The vial was then agitated to disperse the emulsion in the propellant. Table 8 below shows the composition (percentage weight-by-weight basis) of the formulations after addition of propellant.

| **Table 7** | | | | | | |
|---|---|---|---|---|---|---|
| **Formulations Prior to the Addition of Propellant** | | | | | | |
| **Ingredient** | **No 16** | **No 17** | **No18** | **No 19** | **No 20** | **No 21** |
| Imiquimod | 5.88 | 5.88 | 5.56 | 5.26 | 5.26 | 1.05 |
| Isostearic acid | 29.41 | 29.41 | 27.78 | 26.32 | 26.32 | 5.26 |
| Benzyl alcohol | 2.35 | 2.35 | 2.22 | 2.11 | 2.11 | 0.42 |
| Polysorbate 60 | 4.00 | 4.00 | 3.78 | 3.58 | 3.58 | 0.72 |
| Sorbitan monostearate | 0.71 | 0.71 | 0.67 | 0.63 | 0.63 | 0.13 |
| Xanthan gum | 0.59 | 0.59 | 0.56 | 0.53 | 1.05 | 1.05 |
| Methylparaben | 0.24 | 0.24 | 0.22 | 0.21 | 0.11 | 0.11 |
| Propylparaben | 0.02 | 0.02 | 0.02 | 0.02 | 0 | 0 |
| Water | 56.80 | 56.80 | 59.20 | 61.35 | 60.95 | 91.26 |

| **Table 8** | | | | | | |
|---|---|---|---|---|---|---|
| **Formulations After the Addition of Propellant** | | | | | | |
| **Ingredient** | **No 16** | **No 17** | **No 18** | **No 19** | **No 20** | **No 21** |
| Imiquimod | 5 | 5 | 5 | 5 | 5 | 1 |
| Isostearic acid | 25 | 25 | 25 | 25 | 25 | 5 |
| Benzyl alcohol | 2 | 2 | 2 | 2 | 2 | 0.4 |
| Polysorbate 60 | 3.4 | 3.4 | 3.4 | 3.4 | 3.4 | 0.68 |
| Sorbitan monostearate | 0.6 | 0.6 | 0.6 | 0.6 | 0.6 | 0.12 |
| Xanthan gum | 0.5 | 0.5 | 0.5 | 0.5 | 1 | 1 |
| Methylparaben | 0.2 | 0.2 | 0.2 | 0.2 | 0.1 | 0.1 |
| Propylparaben | 0.02 | 0.02 | 0.02 | 0.02 | 0 | 0 |
| Water | 48.28 | 48.28 | 53.28 | 58.28 | 57.9 | 86.7 |
| 50:50 Propane:butane | 15 | 0 | 10 | 5 | 0 | 0 |
| 10:90 Propane:butane | 0 | 15 | 0 | 0 | 5 | 5 |

### Example 1

Male CD hairless rats (Charles River Laboratories, Wilmington, MA) weighing between 225-250 grams (approx. 8-10 weeks of age) are acclimated to neck collars for two consecutive days prior to dosing. On the day of the study, the rats are collared to prevent oral ingestion of the formulation, then treated topically with 45-47 mg of formulation from either Table 3 or Table 8 over an area of 6 approximately cm² at the right, lower back. The formulation is massaged into the skin using a nitrile-gloved finger for approximately 1-3 minutes. The rats are individually housed and remain collared for six hours.

After six hours, blood is collected by cardiac puncture under CO₂ anesthesia. The blood is allowed to clot at room temperature for approximately 20 minutes, then the serum is separated from the clot by centrifugation (Beckman Coulter Allegra 21R, S4180 horizontal rotor, Beckman Coulter, Inc. Fullerton, CA) at 2000 rpm for 10 minutes under refrigeration. The resulting serum is stored at -20°C until analyzed for TNF-α and MCP-1 concentrations.

The animals are sacrificed and three 8 mm punch biopsies are obtained from each of two sites of each animal: the treatment site and opposite flank (left, lower back). The biopsy tissues are weighed and placed in a sealed 1.8 mL cryovial and flash frozen in liquid N₂. The frozen dermal tissue is suspended in 1.0 mL of RPMI medium (Protide Pharmaceuticals, St. Paul, MN) 10% fetal bovine serum, 2 mM L-glutamine, 1% penicillin/streptomycin, 5 x 10⁻⁵M 2-mercaptoethanlo and 1% protease inhibitor cocktail set III (Calbiochem/EMD Biosciences, San Diego, CA). The dermal tissue is homogenized on ice using a TISSUE TEAROR (Biospec Products, Inc. Bartlesville, OK) for approximately one minute. The dermal tissue supernatants are centrifuged (Beckman Coulter Allegra 21R, S4180 horizontal rotor) at 4800 rpm for 10 minutes under refrigeration to pellet cell debris. The supernatants are collected and stored at -20°C until analyzed for TNF-α and MCP-1 concentrations.

TNF-α and MCP-1 concentrations are assayed by ELISA (TNF-α, BD Pharmingen, San Diego, CA; MCP-1, Biosource International, Camarillo, CA) according to manufacturer's specifications. ELISA plates are read on a SpectraMax microassay plate reader (Molecular Devices Corp., Sunnyvale, CA) and software SOFTMAX PRO is utilized for curve fitting of absorbance readings. Results for serum concentrations of TNF-α and MCP-1 are expressed in picograms/milliliter (pg/mL) for serum. Results for dermal tissue concentrations are expressed as pg per 200 mg of tissue.

## Claims

1. A pharmaceutical foam formulation comprising a therapeutically effective amount of imiquimod present in an amount of 0,1% to 8% by weight based on the total weight of the formulation, isostearic acid in an amount of 1% to 40% by weight based on the total weight of the formulation, a preservative system selected from the group consisting of methylparaben and propylparaben, in an amount of 0,01% to 3% by weight based on the total weight of the formulation, sorbitan monostearate in an amount of 2.5% to 10% by weight based on the total weight of the formulation a 50:50 combination of propane and butane propellant in an amount of 10% to 25% by weight based on the total weight of the formulation .

2. A formulation according to claim 1 further comprising water.

3. A formulation according to any one of claims 1 to 2 further comprising a viscosity enhancing agent in an amount of 0.1 % to 10% by weight based on the total weight of the formulation.

4. A formulation according to any one of claims 1 to 3 further comprising an emollient selected from the group consisting of cetyl alcohol, stearyl alcohol, white petrolatum or mixtures thereof present in an amount of 1,0% to 30%.

5. A formulation according to any one of claims 1 to 4 further comprising glycerin.

6. A formulation according to any one of claims 2 to 5 **characterized in that** the formulation is an oil-in-water emulsion.

7. A packaged formulation comprising a formulation of any one of claims 1 to 6 enclosed in an aerosol vial.

8. A packaged formulation according to claim 7 **characterized in that** the aerosol vial is equipped with a metering valve.

9. A packaged formulation according to claim 7 **characterized in that** the aerosol vial is equipped with a continuous valve.

10. Use of a formulation according to any one of claims 1 to 6 for the production of a medicament for the treatment of actinic keratosis.

11. Use of a formulation according to any one of claims 1 to 6 for the production of a medicament for the treatment of basal cell carcinoma.

12. Use of a formulation according to any one of claims 1 to 6 for the production of a medicament for the treatment of anogential warts.

13. Use of a formulation according to any one of claims 1 to 6 for the production of a medicament for the treatment of photodamaged skin.

14. Use of a formulation according to any one of claims 1 to 6 for the production of a medicament for the treatment of cervical dysplasia.

15. Use of a formulation according to any one of claims 1 to 6 for the production of a medicament for the treatment of viral diseases, especially herpes.

16. Use of a formulation according to any one of claims 1 to 6 for the production of a medicament for the treatment of cutaneous metastases.

17. Use of a formulation according to any one of claims 1 to 6 for the production of a medicament for the treatment of dermal lesions caused by envenomation.

18. Use of a formulation according to any one of claims 1 to 6 for the production of a medicament for the treatment of keratoacanthoma.

## Patentansprüche

1. Pharmazeutische Schaumformulierung, umfassend eine therapeutisch wirksame Menge von Imiquimod, das in einer Menge von 0,1 Gew.-% bis 8 Gew.-%, bezogen auf das Gesamtgewicht der Formulierung, vorhanden ist, Isostearinsäure in einer Menge von 1 Gew.-% bis 40 Gew.-%, bezogen auf das Gesamtgewicht der Formulierung, ein Konservierungssystem, das ausgewählt ist aus der Gruppe bestehend aus Methylparaben und Propylparaben, in einer Menge von 0,01 Gew.-% bis 3 Gew.-%, bezogen auf das Gesamtgewicht der Formulierung, Sorbitanmonostearat in einer Menge von 2,5 Gew.-% bis 10 Gew.-%, bezogen auf das Gesamtgewicht der Formulierung, eine 50:50-Kombination von Propan- und Butan-Treibmittel in einer Menge von 10 Gew.-% bis 25 Gew.-%, bezogen auf das Gesamtgewicht der Formulierung.

2. Formulierung nach Anspruch 1, weiterhin umfassend Wasser.

3. Formulierung nach einem der Ansprüche 1 bis 2, weiterhin umfassend ein viskositätserhöhendes Mittel in einer Menge von 0,1 Gew.-% bis 10 Gew.-%, bezogen auf das Gesamtgewicht der Formulierung.

4. Formulierung nach einem der Ansprüche 1 bis 3, weiterhin umfassend einen Weichmacher, der ausgewählt ist aus der Gruppe bestehend aus Cetylalkohol, Stearylalkohol, weißem Petrolatum oder Mischungen davon, der in einer Menge von 1,0 % bis 30 % vorhanden ist.

5. Formulierung nach einem der Ansprüche 1 bis 4, weiterhin umfassend Glycerin.

6. Formulierung nach einem der Ansprüche 2 bis 5, **dadurch gekennzeichnet, dass** die Formulierung eine Öl-in-Wasser-Emulsion ist.

7. Verpackte Formulierung, umfassend eine Formulierung nach einem der Ansprüche 1 bis 6 eingeschlossen in einem Aerosolbehälter.

8. Verpackte Formulierung nach Anspruch 7, **dadurch gekennzeichnet, dass** der Aerosolbehälter mit einem Dosierventil versehen ist.

9. Verpackte Formulierung nach Anspruch 7, **dadurch gekennzeichnet, dass** der Aerosolbehälter mit einem Stetigventil versehen ist.

10. Verwendung einer Formulierung nach einem der Ansprüche 1 bis 6 zur Herstellung eines Medikaments zur Behandlung von aktinischer Keratose.

11. Verwendung einer Formulierung nach einem der Ansprüche 1 bis 6 zur Herstellung eines Medikaments zur Behandlung eines Basalzellkarzinoms.

12. Verwendung einer Formulierung nach einem der Ansprüche 1 bis 6 zur Herstellung eines Medikaments zur Behandlung von Anogenitalwarzen.

13. Verwendung einer Formulierung nach einem der Ansprüche 1 bis 6 zur Herstellung eines Medikaments zur Behandlung von lichtgeschädigter Haut.

14. Verwendung einer Formulierung nach einem der Ansprüche 1 bis 6 zur Herstellung eines Medikaments zur Behandlung von Zervixdysplasie.

15. Verwendung einer Formulierung nach einem der Ansprüche 1 bis 6 zur Herstellung eines Medikaments zur Behandlung von viralen Erkrankungen, insbesondere von Herpes.

16. Verwendung einer Formulierung nach einem der Ansprüche 1 bis 6 zur Herstellung eines Medikaments zur Behandlung von Hautmetastasen.

17. Verwendung einer Formulierung nach einem der Ansprüche 1 bis 6 zur Herstellung eines Medikaments zur Behandlung von durch Vergiftung verursachten Hautläsionen.

18. Verwendung einer Formulierung nach einem der Ansprüche 1 bis 6 zur Herstellung eines Medikaments zur Behandlung eines Keratoakanthoms.

## Revendications

1. Formulation de mousse pharmaceutique comprenant une quantité thérapeutiquement efficace d'imiquimod présent dans une quantité de 0,1 % à 8 % en poids sur la base du poids total de la formulation, de l'acide isostéarique dans une quantité de 1 % à 40 % en poids sur la base du poids total de la formulation, un système conservateur choisi dans le groupe constitué par le méthylparabène et le propylparabène, dans une quantité de 0,01 % à 3 % en poids sur la base du poids total de la formulation, du monostéarate de sorbitane dans une quantité de 2,5 % à 10 % en poids sur la base du poids total de la formulation, un mélange 50:50 de gaz propulseur propane et butane dans une quantité de 10 % à 25 % en poids sur la base du poids total de la formulation.

2. Formulation selon la revendication 1, comprenant en outre de l'eau.

3. Formulation selon l'une quelconque des revendications 1 ou 2, comprenant en outre un agent renforçant la viscosité dans une quantité de 0,1 % à 10 % en poids sur la base du poids total de la formulation.

4. Formulation selon l'une quelconque des revendications 1 à 3, comprenant en outre un émollient choisi dans le groupe constitué par l'alcool cétylique, l'alcool stéarylique, la vaseline blanche ou leurs mélanges, présent dans une quantité de 1,0 % à 30 %.

5. Formulation selon l'une quelconque des revendications 1 à 4, comprenant en outre de la glycérine.

6. Formulation selon l'une quelconque des revendications 2 à 5, **caractérisée en ce que** la formulation est une émulsion huile dans l'eau.

7. Formulation conditionnée comprenant une formulation de l'une quelconque des revendications 1 à 6 contenue dans un flacon aérosol.

8. Formulation conditionnée selon la revendication 7, **caractérisée en ce que** le flacon aérosol est équipé d'une valve doseuse.

9. Formulation conditionnée selon la revendication 7, **caractérisée en ce que** le flacon aérosol est équipé d'une valve continue.

10. Utilisation d'une formulation selon l'une quelconque des revendications 1 à 6 pour la production d'un médicament pour le traitement de la kératose actinique.

11. Utilisation d'une formulation selon l'une quelconque des revendications 1 à 6 pour la production d'un médicament pour le traitement d'un carcinome basocellulaire.

12. Utilisation d'une formulation selon l'une quelconque des revendications 1 à 6 pour la production d'un médicament pour le traitement de verrues génitales.

13. Utilisation d'une formulation selon l'une quelconque des revendications 1 à 6 pour la production d'un médicament pour le traitement de la peau endommagée par le soleil.

14. Utilisation d'une formulation selon l'une quelconque des revendications 1 à 6 pour la production d'un médicament pour le traitement de la dysplasie du col utérin.

15. Utilisation d'une formulation selon l'une quelconque des revendications 1 à 6 pour la production d'un médicament pour le traitement de maladies virales, notamment l'herpès.

16. Utilisation d'une formulation selon l'une quelconque des revendications 1 à 6 pour la production d'un médicament pour le traitement de métastases cutanées.

17. Utilisation d'une formulation selon l'une quelconque des revendications 1 à 6 pour la production d'un médicament pour le traitement de lésions dermatologiques causées par envenimation.

18. Utilisation d'une formulation selon l'une quelconque des revendications 1 à 6 pour la production d'un médicament pour le traitement du kératoacanthome.
